# EUROPÄISCHE PATENTSCHRIFT

(11) **EP 3 607 931 B1**
(45) Veröffentlichungstag und Bekanntmachung des Hinweises auf die Patenterteilung: **29.03.2023**
(21) Anmeldenummer: 18187673.1
(22) Anmeldetag: 07.08.2018
(51) Int. Cl.: A61J 3/07

(54) **KAPSELVERSCHLIESSEINRICHTUNG ZUM VERSCHLIESSEN ZWEITEILIGER KAPSELN**
CAPSULE CLOSING DEVICE FOR CLOSING TWO-PART CAPSULES
DISPOSITIF DE FERMETURE DE CAPSULE PERMETTANT DE FERMER DES CAPSULES EN DEUX PARTIES

(43) Veröffentlichungstag der Anmeldung: 12.02.2020
(73) Patentinhaber: Harro Höfliger Verpackungsmaschinen GmbH, 71573 Allmersbach im Tal (DE)
(72) Erfinder: Weigel, Marco, 71573 Allmersbach im Tal (DE)
(74) Vertreter: Zurhorst, Stefan

(56) Entgegenhaltungen:
- WO-A1-2005/084608
- JP-B2- 3 419 826
- US-A- 4 615 165
- US-A1- 2008 141 621
- US-A1- 2011 088 355

## Beschreibung

Die Erfindung betrifft eine Kapselverschließeinrichtung zum Verschließen zweiteiliger Kapseln mit den Merkmalen nach dem Oberbegriff des Anspruchs 1.

Eine ähnliche Einrichtung zeigt die Druckschrift US 4,615,165.

Insbesondere im Pharmabereich, aber auch im Bereich von Nahrungsergänzungsmitteln oder dgl. werden schluckbare Kapseln eingesetzt, in deren Innenraum Wirkstoffpräparate oder dgl. eingefüllt sind. Derartige Kapseln sind zweiteilig ausgeführt und bestehen aus einem Kapselunterteil sowie einem darauf aufgesteckten Kapseloberteil. Verbreitete Kapselmaterialien sind Hartgelatine, HPMC (Hydroxylpropylmethylcellulose) oder dgl.

Leerkapseln werden für die Befüllung im lose zusammengesteckten Zustand angeliefert und einer Kapselverschließeinrichtung zugeführt. Diese umfasst eine Kapseloberteilaufnahme und eine Kapselunterteilaufnahme, wobei die lose zusammengesteckte Leerkapsel zunächst in der Kapseloberteilaufnahme zu liegen kommt. Hiervon ausgehend wird das Kapselunterteil aus dem Kapseloberteil beispielsweise mittels Unterdruck herausgezogen und in die Kapselunterteilaufnahme eingeführt. In der Kapselunterteilaufnahme findet die Befüllung des Kapselunterteils statt. Hieran anschließend wird das Kapselunterteil beispielsweise mittels eines Stempels zur Kapseloberteilaufnahme gedrückt und dort in das Kapseloberteil eingeführt. Im praktischen Betrieb einer derartigen Kapselverschließeinrichtung sind während des Öffnens der Kapsel hohe Trennkräfte erforderlich. Derartige Trennkräfte werden durch ein auf das Kapselunterteil wirkendes Vakuum gebildet. Hat sich das Kapselunterteil von dem Kapseloberteil gelöst, wird das Kapselunterteil durch das anliegende, hohe Vakuum derart schnell in die Kapselunterteilaufnahme befördert, dass ein sogenanntes Kapselspringen erfolgen kann. Das Kapselspringen tritt dann auf, wenn das Kapselunterteil unzentriert auf die Kapselunterteilaufnahme trifft und ein Rückimpuls das Kapselunterteil aus der Kapselunterteilaufnahme herausstößt. Ferner kann bei derartig hohen Trennkräften auch der Kapselboden des Kapselunterteils reißen oder beim Aufschlagen auf die Kapselunterteilaufnahme brechen. Da auch beim Verschließen der Kapsel hohe Kräfte notwendig sind, kann durch den am Boden des Kapselunterteils angreifenden Stempel der Boden eingedrückt werden oder sogar brechen.

Der Erfindung liegt die Aufgabe zugrunde, eine gattungsgemäße Kapselverschließeinrichtung derart weiterzubilden, dass ihre Betriebssicherheit gesteigert ist.

Diese Aufgabe wird durch eine Kapselverschließeinrichtung mit den Merkmalen des Anspruchs 1 gelöst.

Der erfindungsgemäßen Kapselverschließeinrichtung liegt die Überlegung zugrunde, dass die notwendigen, hohen Kräfte zum Trennen und zum Verschließen der Kapsel auf einem fehlenden Druckausgleich basieren. Beim Trennen der Kapsel stützt sich das Kapseloberteil entlang seiner Kapselkante auf dem Auflageabsatz der Kapseloberteilaufnahme ab. Das Kapselunterteil liegt bündig an der Einführbohrung der Kapseloberteilaufnahme an. Folglich begrenzen das Kapseloberteil und das Kapselunterteil einen Innenraum, dessen Volumen sich mit Abwärtsbewegung des Kapselunterteils in Richtung der Kapselunterteilaufnahme erweitert. Dabei bleibt der Innenraum zumindest anfänglich im Wesentlichen luftdicht verschlossen. Demnach bildet sich im Innenraum zwischen Kapseloberteil und Kapselunterteil ein Unterdruck, der das Trennen der Kapsel erschwert.

Um die hohen Trennkräfte zu mindern weist die Kapseloberteilaufnahme an ihrer Innenseite an der Einführbohrung mindestens eine Entlüftungsbohrung auf. Vorzugsweise weist die Kapseloberteilaufnahme an ihrer Innenseite an der Einführbohrung mindestens zwei, insbesondere drei Entlüftungsbohrungen auf. Durch die Entlüftungsbohrung kann beim Öffnen der Kapsel ausreichend Luft in den Innenraum zwischen Kapseloberteil und Kapselunterteil nachströmen, so dass ein Druckausgleich gewährleistet wird. Es entsteht kein oder allenfalls ein nur geringer Unterdruck im Innenraum. Mit zunehmender Anzahl an Entlüftungsbohrungen in der Kapseloberteilaufnahme kann der Druckausgleich schneller erfolgen. Das Kapselunterteil lässt sich leichter von dem Kapseloberteil trennen. Wegen der reduzierten Trennkräfte wird das schließlich gelöste Kapselunterteil mit weniger Wucht in die Kapselunterteilaufnahme hinein gesaugt, wodurch ein Kapselspringen oder auch andere Beschädigungen an der Kapsel vermieden werden können.

Der Druckausgleich erfolgt ebenfalls beim Schließen der Kapsel. Wenn das Kapselunterteil durch die Kapseloberteilaufnahme in das Kapseloberteil geschoben wird, reduziert sich das Volumen des Innenraums zwischen Kapselunterteil und Kapseloberteil. Die überschüssige Luft entweicht durch die Entlüftungsbohrung, wodurch ein Überdruck beim Verschließen der Kapsel vermieden werden kann. Demnach wirkt kein Überdruck der Schließkraft an dem Kapselunterteil entgegen. Die Kräfte können dadurch beim Verschließen der Kapsel verringert werden, so dass etwaige Beschädigungen an der Kapsel durch zu hohe Schließkräfte vermieden werden können.

Vorzugsweise verläuft die mindestens eine Entlüftungsbohrung von einer Außenseite der Kapseloberteilaufnahme zur Innenseite der Kapseloberteilaufnahme und mündet in die Einführbohrung der Kapseloberteilaufnahme. Dadurch kann beim Öffnen der Kapsel die Luft in die Kapseloberteilaufnahme nachströmen oder beim Schließen der Kapsel aus der Kapseloberteilaufnahme entweichen. Dadurch ist ein Druckausgleich in der Kapseloberteilaufnahme insbesondere im Innenraum zwischen dem Kapseloberteil und dem Kapselunterteil mit der Umgebung sichergestellt.

Der Auflageabsatz der Kapselverschließeinrichtung weist typischerweise eine umlaufende Innenkante auf. In einem solchen Fall bildet die mindestens eine Entlüftungsbohrung vorteilhaft eine Unterbrechung dieser Innenkante. Demnach mündet die Entlüftungsbohrung genau an der Position in die Kapseloberteilaufnahme, an welcher sich das Kapseloberteil und das Kapselunterteil in verschlossenem Zustand überlappen. Wird das Kapselunterteil aus dem Kapseloberteil herausgezogen, kann der Druckausgleich im Innenraum bereits zu einem frühestmöglichen Zeitpunkt erfolgen, wenn sich Kapselunterteil und Kapseloberteil nicht mehr überlappen. Beim Verschließen der Kapsel kann der Druckausgleich über einen möglichst langen Zeitraum erfolgen, bis sich eben Kapselunterteil und Kapseloberteil wieder überlappen und in sich einen luftdicht abgeschlossenen Innenraum begrenzen.

Die Kapselverschließeinrichtung umfasst bevorzugt eine mit der Entlüftungsbohrung verbundene Absaugeinrichtung zur Absaugung von Staubpartikeln an der Kapsel. Dadurch kann die Kapsel frühzeitig von Staubpartikeln gereinigt werden, die sich beispielsweise beim Abfüllvorgang von Füllgut auf der Kapsel niedergelegt haben oder beim Schließvorgang aufgewirbelt wurden.

Ausführungsbeispiele der Erfindung werden im Folgenden anhand der Zeichnung erläutert. Es zeigen:
- Fig. 1: in einer schematischen, teilweise geschnittenen Seitenansicht eine zweiteilige Kapsel,
- Fig. 2: in einer Längsschnittdarstellung eine erfindungsgemäße Kapselverschließeinrichtung,
- Fig. 3: in einer Schnittdarstellung die Kapseloberteilaufnahme,
- Fig. 4: in perspektivischer Darstellung die Kapseloberteilaufnahme.

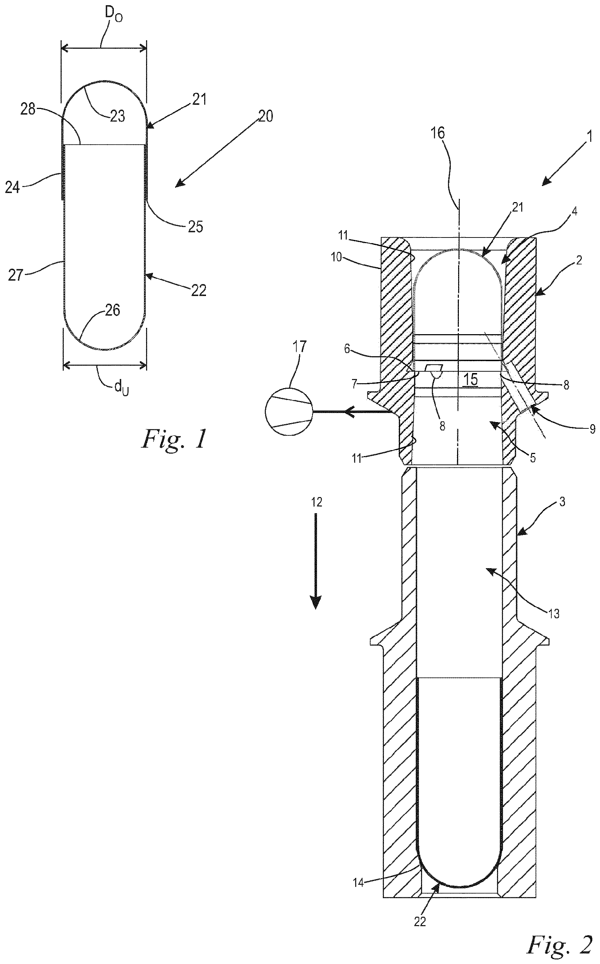

Fig. 1 zeigt in einer schematischen, teilweise geschnittenen Darstellung eine zweiteilige Kapsel 20, wie sie beispielsweise im Pharmabereich oder im Bereich von Nahrungsergänzungsmitteln eingesetzt wird, und die im befüllten Zustand ein Wirkstoffpräparat enthält. Im fertig befüllten und verschlossenen Zustand ist sie zum Verschlucken durch eine Person vorgesehen. Als Kapselmaterial kommen verschiedene Materialen wie Hartgelatine, HPMC oder dgl. in Betracht, welches sich nach dem Verschlucken auflöst und den Kapselinhalt freigibt.

Die zweiteilige Kapsel 1 ist aus einem Kapseloberteil 21 und einem Kapselunterteil 22 zusammengesetzt, wobei das Kapselunterteil 22 mit einem Unterteil-Nenndurchmesser du in die offene Seite des Kapseloberteils 21 mit einem größeren Oberteil-Nenndurchmesser Do eingeschoben ist. Das Kapseloberteil 21 besteht in üblicher Bauform aus einer halbkugeligen Kalotte 23, an die sich ein Zylinderabschnitt 24 anschließt. An seiner offenen Seite ist das Kapseloberteil 21 durch eine umlaufende Kapselkante 25 des Zylinderabschnitts 24 begrenzt. Das Kapselunterteil 22 ist analog zum Kapseloberteil 21 aufgebaut und besteht aus einer halbkugeligen Kalotte 26, an die sich ein Zylinderabschnitt 27 anschließt. An seiner dem Kapseloberteil 21 zugewandten und dort eingeführten Seite ist das Kapselunterteil 22 mit einer umlaufenden Kapselkante 28 des Zylinderabschnitts 27 begrenzt, wobei im zusammengesteckten Zustand von Kapseloberteil 21 und Kapselunterteil 22 ein Teil des Zylinderabschnitts 27 samt seiner umlaufenden Kapselkante 28 innerhalb des Zylinderabschnitts 24 des Kapseloberteils 21 zu liegen kommt.

Fig. 2 zeigt in einer Längsschnittdarstellung eine erfindungsgemäße Kapselverschließeinrichtung 1, die eine Kapseloberteilaufnahme 2 und eine Kapselunterteilaufnahme 3 umfasst. In der Kapselverschließeinrichtung ist eine Kapselverschließanordnung mit einem Kapseloberteil 21 und einem Kapselunterteil 22 einer Kapsel 20 angeordnet.

Leere Kapseln 20 nach Fig. 1 werden in einem Zustand angeliefert, bei denen die Kapselunterteile 22 in jeweils zugeordnete Kapseloberteile 21 lose eingesteckt sind. In diesem Zustand wird eine einzelne Kapsel 20 in die Kapselverschließeinrichtung nach Fig. 2 von oben entsprechend einem Pfeil 12 derart eingeführt, dass sie innerhalb der Kapseloberteilaufnahme 2 zu liegen kommen.

Die Kapseloberteilaufnahme 2 weist eine Aufnahmebohrung 4 zur Aufnahme des Kapseloberteils 21 auf und geht in Richtung zur Kapselunterteilaufnahme 3 in eine koaxial angeordnete Einführbohrung 5 über. Die Einführbohrung 5 ist in ihrem Durchmesser gegenüber dem Durchmesser der Aufnahmebohrung 4 verringert, so dass am Übergang zwischen der Aufnahmebohrung 4 zur Einführbohrung 5 ein umlaufender Auflageabsatz 6 gebildet ist. Der Auflageabsatz 6 begrenzt die Aufnahmebohrung 4 nach unten und ist ebenso wie die Einführbohrung 5 gegenüber der Aufnahmebohrung 4 verengt. Die Kapsel 20 wird derart entsprechend dem Pfeil 12 in die Kapseloberteilaufnahme 2 eingeführt, dass das Kapseloberteil 21 mit seinem Zylinderabschnitt 24 in der Aufnahmebohrung 5 zu liegen kommt, während die umlaufende Kapselkante 25 des Kapseloberteils 21 auf dem Auflageabsatz 6 aufliegt. Der Auflageabsatz 6 hat einen gegenüber der Aufnahmebohrung 4 verringerten Innendurchmesser, durch den das Kapselunterteil 22 hindurchführbar ist.

Unterhalb der Kapseloberteilaufnahme 2 ist die Kapselunterteilaufnahme 3 koaxial positioniert und umfasst eine Aufnahmebohrung 13. Beispielsweise mittels Vakuum oder dgl. wird das in die Einführbohrung 5 ragende Kapselunterteil 22 aus dem Kapseloberteil 21 in die Aufnahmebohrung 13 der Kapselunterteilaufnahme 3 in Richtung des Pfeils 12 herausgezogen, bis es mit seiner nach unten weisenden Kalotte 26 auf einem gegenüber der Aufnahmebohrung 13 im Durchmesser verringerten Absatz 14 zu liegen kommt. Hierbei wird der Zylinderabschnitt 27 des Kapselunterteils 22 durch die Umfangswand der Aufnahmebohrung 14 gestützt.

Das Kapselunterteil 22 wird mit dem nicht dargestellten Wirkstoffpräparat befüllt, während es in der Kapselunterteilaufnahme 3 gehalten ist. Nach erfolgter Befüllung erfolgt ein Verschließen der Kapsel 20 mittels der Kapselverschließeinrichtung 1, indem das Kapselunterteil 21 beispielsweise mittels eines nicht dargestellten Stempels entgegen der Richtung des Pfeils 12 nach oben durch die Aufnahmebohrung 14 und die Einführbohrung 5 hindurch in das Kapseloberteil 21 hineingedrückt wird. Dabei wird eine Gegenkraft auf das Kapseloberteil 21 zur räumlichen Fixierung desselben in Richtung des Pfeils 12 beispielsweise mittels eines ebenfalls nicht dargestellten Stempels ausgeübt. Während des Verschließvorgangs gleitet die umlaufende Kapselkante 28 des Kapselunterteils 22 radial innenseitig der umlaufenden Kapselkante 25 des Kapseloberteils 21 in dessen Zylinderabschnitt 24 hinein.

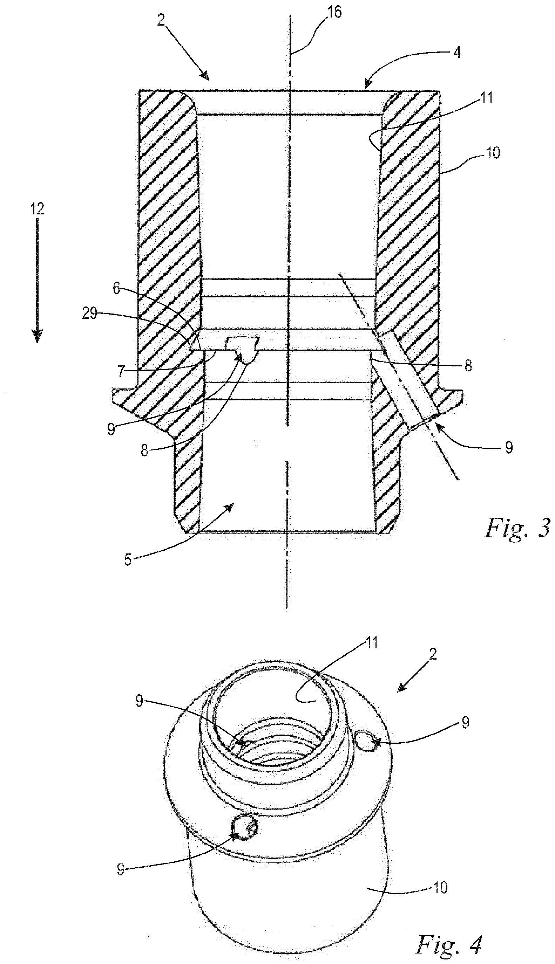

In Fig. 3 ist in einer vergrößerten Schnittdarstellung die Kapseloberteilaufnahme 2 nach der Fig. 2 gezeigt. Die Aufnahmebohrung 4 verläuft in Richtung des Pfeils 12 konisch bis in etwa zum Auflageabsatz 6 zu. Dadurch ist die Aufnahmebohrung 4 trichterförmig ausgebildet und erleichtert das Einfädeln der Kapsel 20 in die Kapseloberteilaufnahme 2. Unmittelbar an dem Auflageabsatz 6 angrenzend ist eine umlaufende Ringnut 29 ausgebildet, die zur Aufnahme von unerwünscht ausgetretenem Füllmaterial dient. Hierdurch wird verhindert, dass die Kapselkante 25 des Kapseloberteils 21 durch das genannte, auf dem Auflageabsatz 6 angesammelte Füllmaterial radial nach innen gedrückt wird. Die Einführbohrung 5 ist entgegen der Pfeilrichtung 12 konisch ausgebildet. Die Einführbohrung 5 verjüngt sich trichterartig in Richtung des Auflageabsatzes 6, wodurch das Einfädeln des Kapselunterteils 22 nach dem Füllvorgang in die Einführbohrung 5 begünstigt wird.

Wie in den Fig. 2 und 3 gezeigt, sind an der Kapseloberteilaufnahme 2 mehrere Entlüftungsbohrungen 9 ausgebildet. Das bevorzugte Ausführungsbeispiel der Kapseloberteilaufnahme 2 weist drei Entlüftungsbohrungen 9 auf (Fig. 4). Es kann jedoch bereits die Ausbildung einer einzigen Entlüftungsbohrung 9 zweckmäßig sein.

Beim Trennen der Kapsel 20 liegt das Kapseloberteil 21 mit seiner Kapselkante 25 bündig an der Innenseite 11 der Aufnahmebohrung 4 an. Ist das Kapselunterteil 22 aus dem Kapseloberteil 22 herausgezogen, liegt die Kapselkante 28 des Kapselunterteils 22 ebenfalls bündig an der Innenseite 11 der Einführbohrung 5 an. Dabei begrenzen Kapseloberteil 21 und Kapselunterteil 22 einen Innenraum 15. Durch die Entlüftungsbohrung 9 wird ein Luftaustausch zwischen dem Innenraum 15 und der Umgebung außerhalb der Kapseloberteilaufnahme 2 ermöglicht. Folglich findet beim Trennen und Schließen der Kapsel 20 ein Druckausgleich statt, so dass ein im Innenraum 15 anliegender Unterdruck oder Überdruck vermieden werden kann.

Die Entlüftungsbohrung 9 verläuft von einer Außenseite 10 zur Innenseite 11 der Kapseloberteilaufnahme 2 und mündet in der Einführbohrung 5. Im Ausführungsbeispiel verläuft die Entlüftungsbohrung 9 geneigt zur Längsachse 16 der Kapseloberteilaufnahme 2, wobei eine andere Ausrichtung der Entlüftungsbohrung 9 zweckmäßig sein kann. Um einen Druckausgleich zu ermöglichen, kann es ausreichend sein, die Entlüftungsbohrung 9 nicht als Durchgangsbohrung, sondern lediglich als Sacklochbohrung auszubilden, und dadurch ein Luft-Reservoir zum Druckausgleich bereitzustellen.

Wie in Fig. 3 gezeigt, verläuft im bevorzugten Ausführungsbeispiel die Entlüftungsbohrung 9 durch den Auflageabsatz 6 und bildet dadurch eine Unterbrechung 8 der im Wesentlichen umlaufenden Innenkante 7 des Auflageabsatzes 8. Es kann auch zweckmäßig sein, die Entlüftungsbohrung 9 an der Innenkante 7 des Auflageabsatzes 8 angrenzend, jedoch zumindest benachbart, in die Einführbohrung 9 mündend auszubilden. Je näher die Entlüftungsbohrung 9 am Trenn- und Schließpunkt der Kapsel 20 liegt, desto früher kann beim Trennen der Kapsel 20 ein Druckausgleich erfolgen, und desto länger hält der Druckausgleich beim Schließen der Kapsel 20 an. Der Trenn- und Schließpunkt der Kapsel 20 liegt im Ausführungsbeispiel im Bereich des Auflageabsatzes 6. Mit der Unterbrechung 8 der Innenkante 7 des Auflageabsatzes 6 liegt die Kapselkante 25 des Kapseloberteils 21 nicht luftdicht an dem Auflageabsatz 6 an, wodurch ein frühestmöglicher Druckausgleich gewährleistet wird.

In der Fig. 4 ist in perspektivischer Darstellung die Kapseloberteilaufnahme 2 nach dem Ausführungsbeispiel der Fig. 2 gezeigt. Die drei Entlüftungsbohrungen 9 sind in Umfangsrichtung der Längsachse 16 der Kapseloberteilaufnahme 2 in einem gleichmäßigen Winkelabstand von 120° verteilt. Es kann auch zweckmäßig sein, die Entlüftungsbohrungen 9 an der Kapseloberteilaufnahme 2 in einem ungleichmäßigen Winkelabstand in Umfangsrichtung der Längsachse 16 auszubilden.

Die erfindungsgemäße Kapselverschließeinrichtung 1 kann in Weiterbildung eine in Fig. 2 nur schematisch angedeutete Absaugeinrichtung 17 umfassen. Die Absaugeinrichtung 17 ist mit der einen oder mit den mehreren Entlüftungsbohrungen 9 verbunden und dient vornehmlich zur Absaugung von an der Kapsel 20 anhaftenden Staubpartikeln. Zur Reinigung der Kapsel 20 ist die Absaugeinrichtung 17 lediglich dann eingeschaltet, wenn die Kapsel 20 nach dem Füllvorgang bereits wieder verschlossen ist. Dadurch wird vermieden, dass der Kapselinhalt durch die Absaugeinrichtung 17 aus dem Kapselunterteil 3 abgesaugt wird. Zudem kann die Absaugeinrichtung 17 auch zur Reinigung der Kapseloberteilaufnahme 2 von Staubpartikeln verwendet werden, die sich beispielsweise auf der Innenseite 11 der Kapseloberteilaufnahme 2 absetzen.

## Patentansprüche

1. Kapselverschließeinrichtung zum Verschließen zweiteiliger Kapseln (20) mit je einem Kapseloberteil (21) und je einem Kapselunterteil (22),
wobei die Kapselverschließeinrichtung (1) eine Kapseloberteilaufnahme (2) und eine Kapselunterteilaufnahme (3) umfasst,
wobei die Kapseloberteilaufnahme (2) eine Aufnahmebohrung (4) und eine koaxial zur Aufnahmebohrung (4) angeordnete Einführbohrung (5) aufweist, und wobei die Kapseloberteilaufnahme (2) an ihrer Innenseite (11) zwischen der Aufnahmebohrung (4) und der Einführbohrung (5) einen Auflageabsatz (6) zur Abstützung des Kapseloberteils (21) aufweist,
**dadurch gekennzeichnet, dass** die Kapseloberteilaufnahme (2) an ihrer Innenseite (11) an der Einführbohrung (5) mindestens eine Entlüftungsbohrung (9) aufweist.

2. Kapselverschließvorrichtung nach Anspruch 1,
**dadurch gekennzeichnet, dass** die Kapseloberteilaufnahme (2) an ihrer Innenseite (11) an der Einführbohrung (5) mindestens zwei, insbesondere drei Entlüftungsbohrungen (9) aufweist.

3. Kapselverschließeinrichtung nach Anspruch 1 oder 2,
**dadurch gekennzeichnet, dass** die mindestens eine Entlüftungsbohrung (9) von einer Außenseite (10) der Kapseloberteilaufnahme (2) zur Innenseite (11) der Kapseloberteilaufnahme (2) verläuft und in die Einführbohrung (5) der Kapseloberteilaufnahme (2) mündet.

4. Kapselverschließvorrichtung nach einem der Ansprüche 1 bis 3,
**dadurch gekennzeichnet, dass** der Auflageabsatz (6) eine umlaufende Innenkante (7) aufweist, und die mindestens eine Entlüftungsbohrung (9) eine Unterbrechung (8) der Innenkante (7) bildet.

5. Kapselverschließeinrichtung nach einem der Ansprüche 1 bis 4,
**dadurch gekennzeichnet, dass** die Kapselverschließeinrichtung (1) eine mit der Entlüftungsbohrung (9) verbundene Absaugeinrichtung zur Absaugung von Staubpartikeln an der Kapsel (20) umfasst.

## Claims

1. Capsule closure device for closing two-piece capsules (20) each having a capsule upper portion (21) and a capsule lower portion (22),
wherein the capsule closure device (1) comprises a capsule upper portion receiving member (2) and a capsule lower portion receiving member (3), wherein the capsule upper portion receiving member (2) has a receiving hole (4) and an introduction hole (5) which is arranged coaxially relative to the receiving hole (4),
and wherein the capsule upper portion receiving member (2) has at the inner side (11) thereof between the receiving hole (4) and the introduction hole (5) a support shoulder (6) for supporting the capsule upper portion (21),
**characterized in that** the capsule upper portion receiving member (2) has at the inner side (11) thereof at the introduction hole (5) at least one ventilation hole (9).

2. Capsule closure device according to Claim 1,
**characterized in that** the capsule upper portion receiving member (2) has at the inner side (11) thereof at the introduction hole (5) at least two, in particular three, ventilation holes (9).

3. Capsule closure device according to Claim 1 or 2,
**characterized in that** the at least one ventilation hole (9) extends from an outer side (10) of the capsule upper portion receiving member (2) to the inner side (11) of the capsule upper portion receiving member (2) and opens in the introduction hole (5) of the capsule upper portion receiving member (2).

4. Capsule closure device according to one of Claims 1 to 3,
**characterized in that** the support shoulder (6) has a peripheral inner edge (7), and the at least one ventilation hole (9) forms an interruption (8) of the inner edge (7).

5. Capsule closure device according to one of Claims 1 to 4,
**characterized in that** the capsule closure device (1) comprises a suction device which is connected to the ventilation hole (9) in order to extract dust particles on the capsule (20).

## Revendications

1. Dispositif de fermeture de capsules pour la fermeture de capsules (20) en deux parties avec respectivement une partie supérieure de capsule (21) et une partie inférieure de capsule (22), le dispositif de fermeture de capsules (1) comprenant un logement de partie supérieure de capsule (2) et un logement de partie inférieure de capsule (3), le logement de partie supérieure de capsule (2) présentant un alésage de logement (4) et un alésage d'introduction (5) agencé coaxialement à l'alésage de logement (4), et le logement de partie supérieure de capsule (2) présentant sur son côté intérieur (11), entre l'alésage de logement (4) et l'alésage d'introduction (5), un épaulement d'appui (6) pour supporter la partie supérieure de capsule (21),
**caractérisé en ce que** le logement de partie supérieure de capsule (2) présente sur son côté intérieur (11), au niveau de l'alésage d'introduction (5), au moins un alésage d'aération (9).

2. Dispositif de fermeture de capsules selon la revendication 1, **caractérisé en ce que** le logement de partie supérieure de capsule (2) présente sur son côté intérieur (11), au niveau de l'alésage d'introduction (5), au moins deux, notamment trois alésages d'aération (9).

3. Dispositif de fermeture de capsules selon la revendication 1 ou 2, **caractérisé en ce que** l'au moins un alésage d'aération (9) s'étend d'un côté extérieur (10) du logement de partie supérieure de capsule (2) vers le côté intérieur (11) du logement de partie supérieure de capsule (2) et débouche dans l'alésage d'introduction (5) du logement de partie supérieure de capsule (2).

4. Dispositif de fermeture de capsules selon l'une quelconque des revendications 1 à 3, **caractérisé en ce que** l'épaulement d'appui (6) présente un bord intérieur périphérique (7), et l'au moins un alésage d'aération (9) forme une discontinuité (8) du bord intérieur (7).

5. Dispositif de fermeture de capsules selon l'une quelconque des revendications 1 à 4, **caractérisé en ce que** le dispositif de fermeture de capsules (1) comprend un dispositif d'aspiration relié à l'alésage d'aération (9) pour l'aspiration de particules de poussière sur la capsule (20).
